# EUROPEAN PATENT APPLICATION

(11) **EP 3 118 303 A1**
(43) Date of publication of application: **18.01.2017**
(21) Application number: 14885341.9
(22) Date of filing: 02.12.2014
(51) Int. Cl.: C12M 1/34

(54) **IMAGING DEVICE, IMAGING SYSTEM, AND INCUBATOR**

(30) Priority: 10.03.2014 JP 2014046327
(71) Applicant: SCREEN Holdings Co., Ltd., Kyoto 602-8585 (JP)
(72) Inventor: IKEFUJI, Kunio, Kyoto-shi Kyoto 602-8585 (JP)
(74) Representative: Kilian Kilian & Partner
(86) International application number: PCT/JP2014/081857
(87) International publication number: WO 2015/136794

(57) **Abstract**

An imaging apparatus comprises: a culture container including an accommodation space for accommodating a sample carrier carrying biological samples in a culture environment for the biological samples and a first transparent part making the accommodation space observable from outside; and an imager that images the biological samples in the accommodation space via the first transparent part.

## Description

### TECHNICAL FIELD

This invention relates to an imaging apparatus and an imaging system for imaging biological samples carried in a sample container under a culture environment and an incubator suitable for these.

### BACKGROUND ART

In medical and bioscience experiments, liquid or gel-like fluid (e.g. culture fluid and culture medium) is poured into each well of a plate-like tool (e.g. called a microplate, a microtiter plate or the like) on which a multitude of recesses, for example, called wells are arranged, and biological samples such as cells are cultured in an incubator. In the case of performing a follow-up observation for seven days after the sowing of the cells, an operation of taking out the microplate from the incubator and returning the microplate after observing and measuring the biological samples in an imaging apparatus is repeated every day. In this case, the culture environment is interrupted by taking out the microplate from the incubator. Thus, an observation result lacks reliability. Accordingly, an incubator in which an imaging apparatus itself is installed in a temperature-controlled room of the incubator is proposed, for example, in patent literature 1.

### [Citation List]

### [Patent Literature]

[pal 1] Japanese Patent No. 5293733

### SUMMARY OF INVENTION

### TECHNICAL PROBLEM

However, the interior of the temperature-controlled room of the incubator is maintained in a culture environment suitable for the culture of biological samples and is severe as an installation environment of the imaging apparatus. For example, a general culture environment is 37°C and highly humid. Thus, a structure endurable against such a culture environment needs to be adopted for the imaging apparatus and a cost increase of the imaging apparatus is unavoidable.

Further, it leads to the capacity enlargement of the incubator to build the imaging apparatus in the temperature-controlled room of the incubator, thereby causing an increase of running cost and a drastic increase of a time required to reach a desired culture environment from a state where the temperature-controlled room of the incubator is open to the atmosphere, i.e. a rise time. This becomes one of main causes for a reduction in productivity.

This invention was developed in view of the above problems and aims to provide a technology capable of imaging biological samples remaining in a culture environment with excellent productivity and at low cost.

### SOLUTION TO PROBLEM

First aspect of the invention is an imaging apparatus. The imaging apparatus comprises: a culture container including an accommodation space for accommodating a sample carrier carrying biological samples in a culture environment for the biological samples and a first transparent part making the accommodation space observable from outside; and an imager that images the biological samples in the accommodation space via the first transparent part.

Second aspect of the invention is an imaging system. The imaging system comprises: a plurality of culture containers each including an accommodation space for accommodating a sample carrier carrying biological samples in a culture environment for the biological samples and a first transparent part making the accommodation space observable from outside; an imager that images the biological samples; and a driver that switches the culture container facing the imager by relatively moving the imager with respect to the plurality of culture containers; wherein the imager images the biological samples in the accommodation space via the first transparent part of the culture container facing the imager every time the culture container facing the imager is switched.

Third aspect of the invention is an incubator for imaging biological samples carried in a sample carrier by an imager of an imaging apparatus in a state where the biological samples are cultured. The incubator comprises: a culture container including an accommodation space for accommodating the sample carrier; and an environment regulator that regulates the interior of the accommodation space to a culture environment for the biological samples; wherein the culture container is provided separately from the imager and including a first transparent part making the accommodation space observable from outside of the culture container.

In the invention thus configured, the accommodation space of the culture container serves as the culture environment and the sample carrier carrying the biological samples is accommodated into the accommodation space. Further, the culture container is provided with the first transparent part and the biological samples carried on the sample carrier accommodated in the accommodation space is imaged by the imager via the first transparent part. That is, the imager is provided separately from the culture container and images the biological samples in the culture container via the first transparent part. Thus, the biological samples under the culture environment can be imaged without arranging the imager in the culture environment. Further, since it is not necessary to build the imager in the culture container, the culture container can be set to have a capacity suitable for the sample carrier and a capacity reduction can be realized as compared to the apparatus described in patent literature 1.

### ADVANTAGEOUS EFFECTS OF INVENTION

According to this invention, since the biological samples are accommodated in the accommodation space regulated to the culture environment and imaged via the first transparent part of the culture container, the biological samples can be imaged while remaining in the culture environment. Further, since the imager is provided outside the culture container, the capacity of the culture container can be set at the one suitable for the sample carrier and the biological samples can be imaged with excellent productivity and at low cost.

### BRIEF DESCRIPTION OF DRAWINGS

FIG. 1 is a diagram schematically showing the configuration of a first embodiment of an imaging apparatus according to the invention.
FIG. 2 is a diagram showing a main configuration of the incubator.
FIGS. 3A and 3B are graphs showing a result of a cell culture test by the incubator of FIG. 2 and a result of a cell culture test by the CO₂ incubator.
FIG. 4 is a view schematically showing the configuration of an incubator used in a second embodiment of the imaging apparatus according to the invention.
FIG. 5 is a diagram schematically showing the configuration of a third embodiment of the imaging apparatus according to the invention.
FIG. 6 is a flow chart showing a time-lapse operation by the imaging apparatus of FIG. 5.
FIG. 7 is a diagram schematically showing the configuration of the fourth embodiment of the imaging apparatus according to the invention.
FIG. 8 is a diagram showing one embodiment of the imaging system according to the invention.

### DESCRIPTION OF EMBODIMENTS

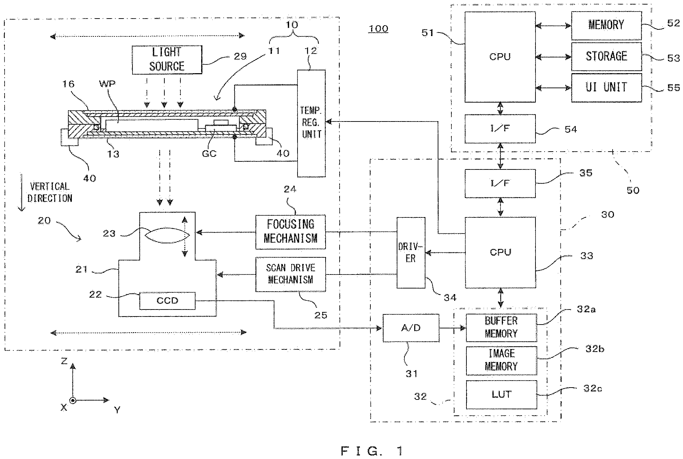

FIG. 1 is a diagram schematically showing the configuration of a first embodiment of an imaging apparatus according to the invention. This imaging apparatus 100 is an apparatus equipped with an incubator 10 and configured to image biological samples cultured in a culture container of the incubator 10, and includes an optical scanning unit 20, a control unit 30, a holder 40 and a host computer 50 besides the incubator 10. Note that XYZ orthogonal coordinate axes with a Z-axis direction as a vertical direction are shown as appropriate to clarify an arrangement relationship of each unit of the apparatus in FIG. 1 and each figure to be described later. Further, if necessary, an arrow side of each coordinate axis in figures is called a positive or (+) side and a side of each coordinate axis opposite to the arrow side in figures is called a negative or (-) side.

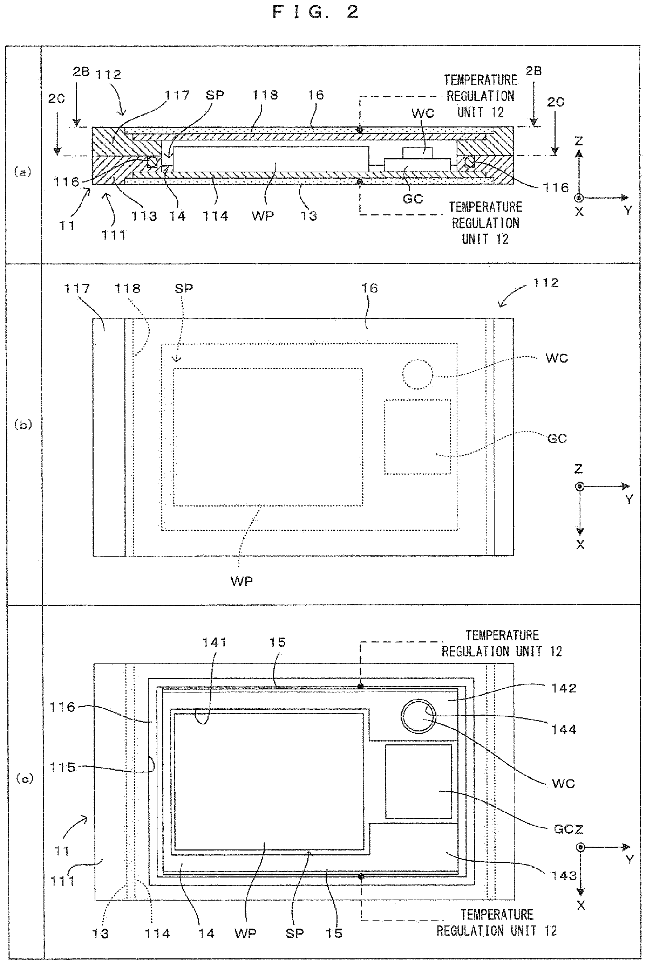

FIG. 2 is a diagram showing a main configuration of the incubator. In FIG. 2, a sectional view showing the structure of the incubator is shown in a section (a), a plan view along line 2B-2B indicated by arrows and a plan view along line 2C-2C indicated by arrows in the sectional view are shown in sections (b) and (c). The incubator 10 includes a culture container 11 and a temperature regulation unit 12. In this culture container, an upper case part 112 is provided openably and closably with respect to a lower case part 111, and an accommodation space SP capable of accommodating a well plate WP, a gas concentration regulator GC and a water container WC is formed in the culture container 11 by closing and integrating the upper case part 112 with the lower case part 111 as shown in FIG. 1. On the other hand, by opening the upper case part 112, it becomes possible to mount and remove the well plate WP, mount the gas concentration regulator GC, exchange the water container WC and the like for the lower case part 111.

The lower case part 111 includes a case main body 113 formed of a resin material such as polyphenylene sulfide resin and a lower surface transparent plate 114. Specifically, the case main body 113 has a frame-like shape with an opening in a central part. The lower surface transparent plate 114 is mounted to close the opening from a (-Z) side in the vertical direction. In this way, the lower surface transparent plate 114 functions as a bottom lid of the lower case part 111 and a lower space area of the accommodation space SP is formed by the case main body 113 and the lower surface transparent plate 114.

A lower surface glass heater 13 is held in close contact with the entire lower surface of this lower surface transparent plate 114. This lower surface glass heater 13 is electrically connected to the temperature regulation unit 12, generates heat upon receiving power corresponding to a temperature command from the control unit 30 from the temperature regulation unit 12 and heats the well plate WP and the accommodation space SP via the lower surface transparent plate 114. Note that the lower surface glass heater 13 is desirably provided to cover 80 [%] or more of the lower surface of the lower case part 111 to increase the temperature of the accommodation space SP from about a room temperature to the temperature of the culture environment and maintain this temperature also during a culturing process.

An inner fixing plate 14 is placed on the upper surface of this lower surface transparent plate 114. The inner fixing plate 14 is substantially C-shaped in a plan view from a vertically upper side as shown in the section (c) of FIG. 2 and provided in a central part with a through hole 141 slightly larger than and similar in shape to a planar shape of the well plate WP. When the well plate WP is placed on the upper surface of the lower surface transparent plate 114 via this central through hole 141, the central through hole 141 of the inner fixing plate 14 restricts a movement of the well plate WP in a horizontal direction. Thus, the stable culturing process can be performed by effectively preventing the well plate WP from moving while the biological samples are cultured in the culture container 11.

Further, a distance between end parts 142, 143 spaced apart from each other in an X direction is set to be about equal to a width of the gas concentration regulator GC in the X direction. When the gas concentration regulator GC is placed on the upper surface of the lower surface transparent plate 114 while being positioned between the end parts 142, 143, the end parts 142, 143 are positioned to sandwich the gas concentration regulator GC from opposite sides in the X direction and restrict a movement of the gas concentration regulator GC. A carbon dioxide concentration regulator, Culture Pal (registered trademark, produced by Mitsubishi Gas Chemical Company, Inc.), can be, for example, used as this gas concentration regulator GC, and is arranged in the culture container 11 in a state where the gas concentration regulator GC is exposed to the accommodation space SP. Thus, a carbon dioxide concentration in the accommodation space SP is regulated to a value suitable for the culture of biological samples by carbon dioxide generated from the gas concentration regulator GC.

Further, the end part 142 of the inner fixing plate 14 is provided with a circular through hole 144 slightly larger than the water container WC having a cylindrical cup shape. When the water container WC is placed on the upper surface of the lower surface transparent plate 114 via this circular through hole 144, the circular through hole 144 of the inner fixing plate 14 restricts a horizontal movement of the water container WC. This water container WC stores water and is arranged in the culture container 11 with the water exposed to the accommodation space SP. Thus, a humidity in the accommodation space SP is kept at a value suitable for the culture of the biological samples by the natural evaporation of the water stored in the water container WC. Note that the shape of the water container WC is arbitrary without being limited to the cylindrical cup shape and the through hole 144 has only to be provided to have a shape corresponding to the shape of the water container WC.

As just described, in this embodiment, the well plate WP, the gas concentration regulator GC and the water container WC can be held on the lower surface transparent plate 114 while being positioned at desired positions by using the inner fixing plate 14 configured as described above.

Further, a film heater 15 is attached to each end surface of the inner fixing plate 14 in the X direction. A Kapton heater insulated with a Kapton (e.g. made of a polyimide material) sheet can be used as the film heater 15. The film heater 15 is electrically connected to the temperature regulation unit 12 similarly to the lower surface glass heater 13, generates heat upon receiving power corresponding to a temperature command from the control unit 30 from the temperature regulation unit 12 and heats the well plate WP and the accommodation space SP via the inner fixing plate 14.

As shown in the sections (a) and (c) of FIG. 2, an annular groove 115 is provided on the upper surface of the case main body 113 to surround the central opening of the case main body 113 in the lower case part 111. An O-ring 116 is press-fitted into this annular groove 115 to keep the airtightness of the accommodation space SP when the lower case part 11 is closed by the upper case part 112.

Similarly to the lower case part 111, this upper case part 112 also includes a frame-shaped case main body 117 and an upper surface transparent plate 118. The upper surface transparent plate 118 is mounted as an upper lid to close a central opening of the case main body 117 from an upper side, i.e. (+Z) side in the vertical direction and a lower space area of the accommodation space SP is formed by the case main body 117 and the upper surface transparent plate 118. Note that, similarly to the lower surface transparent plate 114, an upper surface glass heater 16 is held in close contact with the entire upper surface of this upper surface transparent plate 118. When power corresponding to a temperature command from the control unit 30 is received from the temperature regulation unit 12, the upper surface glass heater 16 generates heat and heats the well plate WP and the accommodation space SP via the upper surface transparent plate 118. Note that, in this embodiment, three types of heaters, i.e. the lower surface glass heater 13, the film heaters 15 and the upper surface glass heater 16 are provided as heating elements and power is fed to each heater by the temperature regulation unit 12. However, a value of the power fed to each heater can be independently controlled. In this way, the temperature in the accommodation space SP is regulated to the temperature suitable for biological samples, e.g. 37°C. Further, the gas concentration regulator GC and the water container WC are provided in the culture container 11 and the carbon dioxide concentration and the humidity of the accommodation space SP are regulated to values suitable for the culture of the biological samples carried in the well plate WP.

In the culture container 11 configured as described above, the accommodation space SP is made openable and closable by an unillustrated opening/closing mechanism. In observing biological samples while culturing them, the well plate WP carrying desired biological samples are accommodated into the accommodation space SP. The well plate WP includes a plurality of, e.g. 96 (12x8 matrix array) wells having a substantially circular cross-section and capable of respectively carrying, for example, a liquid-state or solid-state culture medium. A diameter and a depth of each well are typically about several [mm]. Note that the size and the number of the wells of the well plate as an object of this imaging apparatus 100 are arbitrary without being limited to these. For example, the well plate may be provided with 384 wells.

The culture container 11 accommodating the well plate WP is set in the holder 40 of the imaging apparatus 100. Then, light (e.g. white light) is irradiated from a light source 29 to the well plate WP via the upper surface glass heater 16 and the upper surface transparent plate 118. The light source 29 is, for example, configured by an LED lamp and arranged above the culture container 11 held in the holder 40.

The optical scanning unit 20 is provided as an imager for optically imaging an imaging object by receiving transmitted light from the imaging object. The optical scanning unit 20 includes an imaging section 21 provided below the culture container 111 held in the holder 40 and having a CCD element 22 as a light receiving element and a convergent optical system 23 for adjusting a magnification of an optical image by the transmitted light, a focusing mechanism 24 for focusing the convergent optical system 23 and a scan drive mechanism 25 for driving the imaging section 21 in a predetermined direction (lateral direction in FIG. 1), for example, by driving a belt.

Light transmitted downward from the bottom surfaces of the wells out of the light irradiated toward the well plate WP from the light source 29 is converged by the convergent optical system 23 and received by the CCD element 22, whereby an optical image is converted into an electrical signal. The focusing mechanism 24 adjusts a focusing position of the optical image focused on the CCD element 22 by driving the convergent optical system 23 according to a control command from the control unit 30. Further, the scan drive mechanism 25 moves the light source 29 and the imaging section 21 integrally within a horizontal plane. Thus, a positional relationship of the light source 29 and the imaging section 29 is fixed.

By relatively moving the CCD element 22, which is a line sensor, in a direction perpendicular to an element arrangement direction of the CCD element 22 with respect to the well plate WP, a scanning movement of the CCD element 22 relative to the well plate WP is realized, whereby a two-dimensional image of the biological samples, which are the contents of the wells, is imaged. Note that the optical scanning unit 20 is controlled by the control unit 30.

The control unit 30 includes an A/D converter 31 for converting an electrical signal output from the CCD element 22 into a luminance value (color density value) according to the amount of the transmitted light received from the samples in the wells, a memory 32 for holding a collection of luminance values of the respective pixels obtained from the samples as image data and storing various pieces of set data, a CPU 33 for functioning as a controller for controlling each unit of the apparatus and a driver 34 for driving the focusing mechanism 24 and the scan drive mechanism 25 according to a control command from the CPU 33. Note that the memory 32 is configured by a ROM, a RAM, a nonvolatile memory or the like, includes a buffer memory 32a for temporarily holding luminance value data output from the A/D converter 31, an image memory 32b for holding multi-gradation image data generated based on the luminance value data and the like, and stores various pieces of reference data such as a reference table (LUT) 32c to be referred to when a gradation correction process is performed.

The control unit 30 configured as just described can communicate with the host computer 50 for controlling the operation of the entire imaging apparatus 100 via an interface unit (I/F) 35. Specifically, the host computer 50 is configured similarly to general personal computers and includes a CPU 51 for performing various arithmetic processings, a memory 52 for temporarily storing control data generated by the operation of the CPU 51, a storage 53 for storing and saving control programs to be executed by the CPU 51 and the interface unit (I/F) 54 for transferring data to and from the control unit 30.

Further, the host computer 50 includes a user interface (UI) unit 55 for receiving various operation inputs from a user and presenting various pieces of information to the user. More specifically, the UI unit 55 includes at least one type of input devices such as operation buttons, a keyboard, a mouse and a touch panel as a receiver for receiving operation inputs from the user. Further, the UI unit 55 includes a display for displaying, for example, an obtained image, a message and the like on a screen as an output unit for presenting information to the user.

Functions of receiving various operation inputs from the user for the operation of the imaging apparatus 100 and presenting an image obtained as a result of the operation to the user are concentrated in the host computer 50. Thus, the control unit 30 has a minimum configuration to cause the optical scanning unit 20 to perform a predetermined operation. As just described, this apparatus is provided with the control unit 30 having a minimum necessary control function for the operation of specific hardware and, on the other hand, configured to perform more general processings by the host computer 50 having general versatility, whereby system cost can be suppressed to be low.

Note that this imaging apparatus 100 is integrally equipped with the incubator 10, the optical scanning unit 20, the control unit 30 and the holder 40 as described above and controlled by the general-purpose host computer 50. Instead of this, all configurations necessary for imaging may be integrated as a unit. In the case of configuring an imaging apparatus by an imaging unit (= light source 29 + optical scanning unit 20) and a host computer, hardware and software resources for the saving of image data and the execution of various analytical processings can be concentrated in the host computer. By doing so, the imaging unit side is sufficient to include only hardware and software minimum necessary for imaging, wherefore system cost can be suppressed.

Next, a time-lapse (follow-up observation) operation by the imaging apparatus 100 configured as described above is described. After preparing the well plate WP in which the culture medium containing biological tissues and cells is poured into the wells, the user places the water container WC storing water, the gas concentration regulator GC and the well plate WP on the upper surface of the lower surface transparent plate 114 while fixing them by the inner fixing plate 14 as shown in the sections (a) and (c) of FIG. 2 with the upper case part 112 of the culture container 11 opened. Then, the culture container 11 is set in the holder 40 of the imaging apparatus 100 after the upper case part 112 is closed to keep the accommodation space SP airtight.

When the user gives a command to the effect of starting a time lapse after the setting of the culture container 11 is completed, the control unit 30 controls each unit of the apparatus in accordance with a program given in advance to perform the culturing process by the incubator 10 and a sample imaging process by the optical scanning unit 20. Specifically, the control unit 30 sends a temperature command to the temperature regulation unit 12 upon receiving the start command. Then, the temperature regulation unit 12 supplies power to each heater 13, 15 and 16 to start temperature regulation in the accommodation space SP. Here, in this embodiment, the culture container 11 is configured to accommodate only elements minimum necessary to establish the culture environment, i.e. the water container WC and the gas concentration regulator GC besides the well plate WP. This makes a capacity or the accommodation space SP, which is a space supposed to form the culture environment in the incubator 10, drastically smaller than temperature-controlled rooms of the incubator described in patent literature 1 and general CO₂ incubators. As a result, a time required to set a desired culture environment temperature, e.g. 37°C from the start of temperature regulation by the heaters 13, 15 and 16 and the temperature regulation unit 12 can be shortened, and productivity can be improved. Further, in this way, not only the temperature is kept constant, but also the culture environment suitable for the culture of the biological samples is regulated by the water stored in the water container WC and carbon dioxide generated from the gas concentration regulator GC.

In this embodiment, the culturing process is continued while the temperature regulation by the heaters 13, 15 and 16 is performed with the culture container 11 held in the holder 40. The control unit 30 performs an operation of reading the biological samples in the culture container 11 at every predetermined time, e.g. every time 24 hours elapse while counting the time elapsed after the pouring of the culture medium into the wells (i.e. sowing of the biological tissues and cells). Specifically, the imaging apparatus 100 performs the reading operation to image the cultured biological samples every time 24 hours elapses. An image signal generated by the CCD element 22 of the imaging section 21 by reading is converted into multi-value original image data by the A/D converter 31 of the control unit 30. Since the original image data at this time includes the influence of a nonlinear sensitivity characteristic of an imaging system, a gradation correction process is applied to the original image data to generate multi-gradation image data having such nonlinearity eliminated in this imaging apparatus 100.

As described above, the well plate WP is accommodated into the accommodation space SP having the culture environment suitable for the culture and the culturing process is performed in the culture container 11 as one constituent element of the incubator 10. Further, in the culture container 11, a member for placing the well plate WP is configured by the lower surface transparent plate 114 and light emitted from the biological samples cultured in the well plate WP is guided to the outside of the culture container 11 via the lower surface transparent plate 114 and incident on the optical scanning unit 20 after being transmitted through the lower surface glass heater 13. That is, although the culture container 11 for culturing the biological samples and the optical scanning unit 20 for imaging the biological samples are separately arranged in this embodiment, the biological samples in the culture container 11 can be imaged by the optical scanning unit 20 via the lower surface transparent plate 114 and the lower surface glass heater 13 while the biological samples are cultured in the accommodation space SP. Thus, the biological samples can be imaged while remaining in the culture environment. Further, since the biological samples can be imaged without moving the culture container 11 after the culture container 11 is set in the holder 40, the follow-up observation can be efficiently and highly reliably performed.

Further, the incubator 10 is incorporated into the imaging apparatus 100 in this embodiment, and this embodiment has the following functions and effects as against the apparatus described in patent literature 1, i.e. the imaging apparatus incorporated into the incubator. Specifically, in this embodiment, it is not necessary to build the optical scanning unit 20 in the culture container 11 and a reduction in the capacity of the culture container 11 is possible. As a result, a time required until the interior of the accommodation space SP accommodating the well plate WP is regulated to the culture environment can be drastically shortened and productivity can be improved. Further, the amount of power required for the temperature regulation of the accommodation space SP and the generation amount of carbon dioxide in the accommodation space SP can be suppressed by a reduction in the capacity of the culture container 11 and the running cost can be reduced.

Further, since illumination light is irradiated to the well plate WP via the upper surface glass heater 16 and the upper surface transparent plate 118 in the above embodiment, the biological samples can be satisfactorily imaged.

Further, since the temperature in the accommodation space SP is regulated using three types of heaters 13, 15 and 16 in this embodiment, this temperature can be regulated to the temperature suitable for the culture environment in a short time. In addition, since the temperature regulation unit 12 is configured to independently control the power supplied to each heater 13, 15 and 16, each heater can be functionally controlled. That is, out of these heaters, the lower surface glass heater 13 can directly heat the well plate WP via the lower surface transparent plate 114 and regulate the temperature of the well plate WP to the temperature suitable for the culture with high accuracy. Further, the upper surface glass heater 16 is arranged above the well plate WP, heats the upper surface of the well plate WP via the upper surface transparent plate 118 and the accommodation space SP and largely contributes to an accuracy improvement of the follow-up observation by effectively preventing the occurrence of dew condensation on an upper part of the well plate WP and the upper surface transparent plate 118. Furthermore, by providing the film heaters 15, the accommodation space SP can be not only heated from the (+) Z direction side and the (-) Z direction side, but also auxiliarily heated from horizontal direction sides, whereby the temperature of the accommodation space SP can be accurately brought closer to the temperature of the culture environment. This contributes to the stabilization of the culture environment.

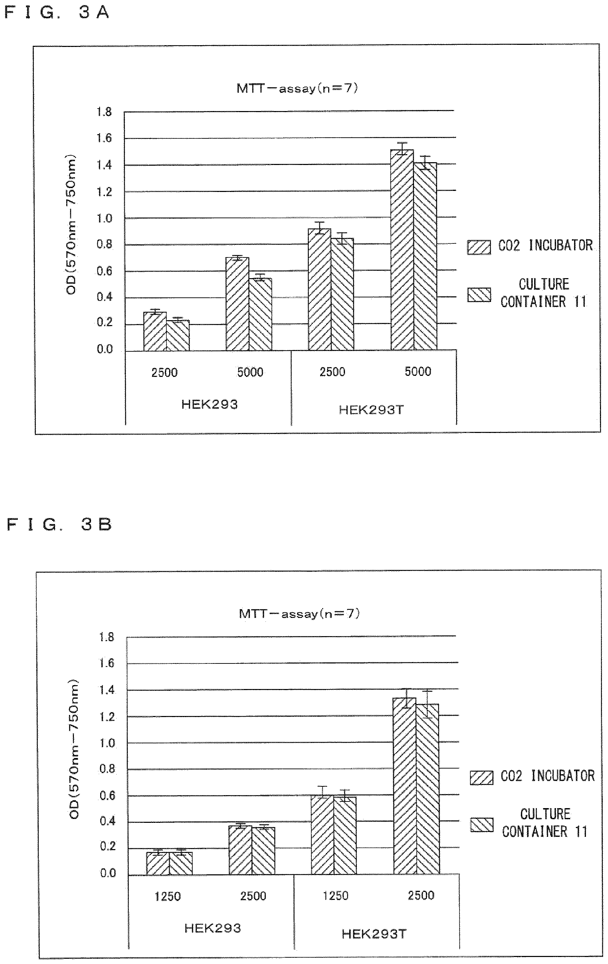

In the above embodiment, the simple incubator 10 different from conventionally generally known CO₂ incubators is adopted in order to construct the culture environment separately from the optical scanning unit 20 in the imaging apparatus 100. Accordingly, to verify whether or not the biological samples can be properly cultured by the incubator 10, the inventors of this application examined a correlation between the number of cells cultured by a CO₂ incubator and that of cells cultured by the simple incubator 10. That verification result is described with reference to FIGS. 3A and 3B.

FIGS. 3A and 3B are graphs showing a result of a cell culture test by the incubator of FIG. 2 and a result of a cell culture test by the CO₂ incubator. The following cell culture test was conducted twice for HEK 293 cells and HEK 293T cells. Out of those, a first test result is shown in FIG. 3A and a second test result is shown in FIG. 3B.

In the first test, two 96-well plates were prepared. In one well plate, 2500 HEK 293 cells per well were sown into n (n = 7 in this embodiment) wells, 5000 HEK 293 cells per well were sown into other seven wells, 2500 HEK 293T cells per well were sown into still other seven wells and 5000 HEKT 293 cells per well were sown into further other seven wells. Further, the cells were similarly sown to the other well plate. Note that cell suspension to be added to each well is set at 100 [µl].

One of the two well plates was incubated for three days in the CO₂ incubator (humidified 5 %-CO₂ atmosphere of 37°C) and the other was incubated for three days in the incubator 10 of FIG. 2. After the culture was completed in this way, the growth of each cell was confirmed by an MTT assay. That is, after an MTT (3-(4,5-di-methylthiazol-2-yl)-2,5-diphenyltetrazolium bromide, yellow tetrazole) reagent was added to each well and caused to react, the growth was confirmed with light absorption amounts at two wavelengths (750 [nm], 570 [nm]) by a spectrophotometer. Out of these two wavelengths, 570 [nm] is a wavelength involved in the action of the MTT reagent, and an OD (Optical Density) at this wavelength indicates the light absorption amount at this wavelength by the action of the MTT reagent. However, the light absorption amount of background is also included. On the other hand, 750 [nm] is a wavelength not involved in the action of the MTT reagent, and an OD at this wavelength indicates the light absorption amount of the background. Accordingly, a value OD (570 nm - 750 nm) obtained by subtracting the OD (750 [nm]) from the OD (570 [nm]) was calculated as the light absorption amount by the action of the MTT reagent and the cell growth by incubation was quantitatively evaluated based on this. That result is compiled in Table 1.

**[Table 1]**

| | Cells | HEK 293 | | HEK 293T | |
|---|---|---|---|---|---|
| | # of cells per well | 2500 | 5000 | 2500 | 5000 |
| Average value | CO₂ incubator | 0.303 | 0.715 | 0.925 | 1.519 |
| | Simple Incubator | 0.245 | 0.561 | 0.848 | 1.416 |
| | Correlation ratio | 0.808 | 0.784 | 0.916 | 0.933 |
| Standard deviation | CO₂ incubator | 0.020 | 0.015 | 0.043 | 0.044 |
| | Simple Incubator | 0.014 | 0.028 | 0.039 | 0.052 |

Note that, in the above tests, seven measurements are made under the same test conditions (cell species, incubation method). In Table 1 and Table 2 to be described later, the "average value" means an average of the ODs (570 nm to 750 nm) measured for seven wells under the same test conditions, the "correlation ratio" means a ratio of the ODs (570 nm to 750 nm) in the case of culturing by the CO₂ incubator for the same number of same cells and the "standard deviation" means a standard deviation of the OD (570 nm to 750 nm).

Further, in the second test, the numbers of the cells are respectively changed to 1250 and 2500 and the cell culture test was conducted with the other test conditions unchanged from those of the first test. That result is compiled in Table 2.

**[Table 2]**

| | Cells | HEK 293 | | HEK 293T | |
|---|---|---|---|---|---|
| | # of cells per well | 1250 | 2500 | 1250 | 2500 |
| Average value | CO₂ incubator | 0.083 | 0.175 | 0.302 | 0.659 |
| | Simple Incubator | 0.083 | 0.174 | 0.296 | 0.638 |
| | Correlation ratio | 0.997 | 0.991 | 0.978 | 0.968 |
| Standard deviation | CO₂ incubator | 0.009 | 0.012 | 0.024 | 0.038 |
| | Simple Incubator | 0.003 | 0.011 | 0.018 | 0.052 |

As understood from these tables, the number of the cells cultured by the simple incubator 10 is 78 % to 100 % of the number of the cells cultured by the CO₂ incubator and the correlation between the both is kept. This indicates that the cells can be satisfactorily cultured as before even if the simple incubator 10 is used instead of the CO₂ incubator and the follow-up observation can be satisfactorily performed by the imaging apparatus 100 equipped with this simple incubator 10.

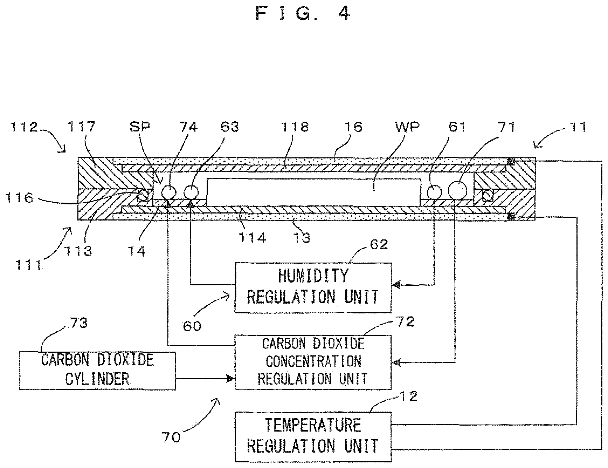

FIG. 4 is a view schematically showing the configuration of an incubator used in a second embodiment of the imaging apparatus according to the invention. This second embodiment largely differs from the first embodiment in the configuration of the incubator 10 equipped in the imaging apparatus 100. Specifically, the humidity and the carbon dioxide concentration of the accommodation space SP of the culture container 11 are simply controlled by arranging the water container WC and the gas concentration regulator GC in the first embodiment, whereas the humidity and the carbon dioxide concentration are highly controlled while being monitored in the second embodiment. The following description is made, centering on points of difference and the same components are denoted by the same reference signs and not described.

In this second embodiment, a humidity regulating mechanism 60 is provided as a humidity regulator for regulating a humidity in an accommodation space SP to a culture environment. This humidity regulating mechanism 60 includes a humidity sensor 61, a humidity regulation unit 62 and a stream supply unit 63. That is, the humidity sensor 61 and the steam supply unit 63 are arranged in the accommodation space SP instead of the water container WC. The humidity sensor 61 measures the humidity in the accommodation space SP and outputs that humidity information to the humidity regulation unit 62. The humidity regulation unit 62 compares the given humidity information with a humidity command value from a CPU 33 and feeds the steam generated by a built-in steam generating mechanism (not shown) to the steam supply unit 63 and supplies it to the accommodation space SP if the measured humidity value is below the humidity command value. As just described, since the accommodation space SP is humidified if necessary while the humidity of the accommodation space SP is monitored in this embodiment, the accommodation space SP can be more accurately kept at the humidity of the culture environment and the culture can be stably performed at the humidity commanded from the CPU 33 of a control unit 30.

Further, in the second embodiment, a gas concentration regulating mechanism 70 is provided as a gas concentration regulator for regulating a carbon dioxide concentration in the accommodation space SP to the culture environment. This gas concentration regulating mechanism 70 includes a carbon dioxide concentration sensor 71, a carbon dioxide concentration regulation unit 72, a carbon dioxide cylinder 73 and a gas supply unit 74. That is, instead of the gas concentration regulator GC, the carbon dioxide concentration sensor 71 and the gas supply unit 74 are arranged in the accommodation space SP. The carbon dioxide concentration sensor 71 measures a carbon dioxide concentration in the accommodation space SP and outputs that carbon dioxide concentration information to the carbon dioxide concentration regulation unit 72. The carbon dioxide concentration regulation unit 72 compares the given carbon dioxide concentration information with a carbon dioxide concentration command value from the CPU 33 and feeds carbon dioxide supplied from the carbon dioxide cylinder 73 to the gas supply unit 74 and supplies it to the accommodation space SP if the measured carbon dioxide concentration value is below the carbon dioxide concentration command value. As just described, since the carbon dioxide is supplied if necessary while monitoring the carbon dioxide concentration of the accommodation space SP in this embodiment, the accommodation space SP can be more accurately kept at the carbon dioxide concentration of the culture environment, and the culture can be stably performed at the carbon dioxide concentration commanded from the CPU 33. Note that although the carbon dioxide cylinder 73 is equipped in the imaging apparatus 100 in this embodiment, carbon dioxide supplied from a supply source of the carbon dioxide may be supplied to the accommodation space SP if the supply source is provided in a factory where the imaging apparatus 100 is installed or a building such as a laboratory.

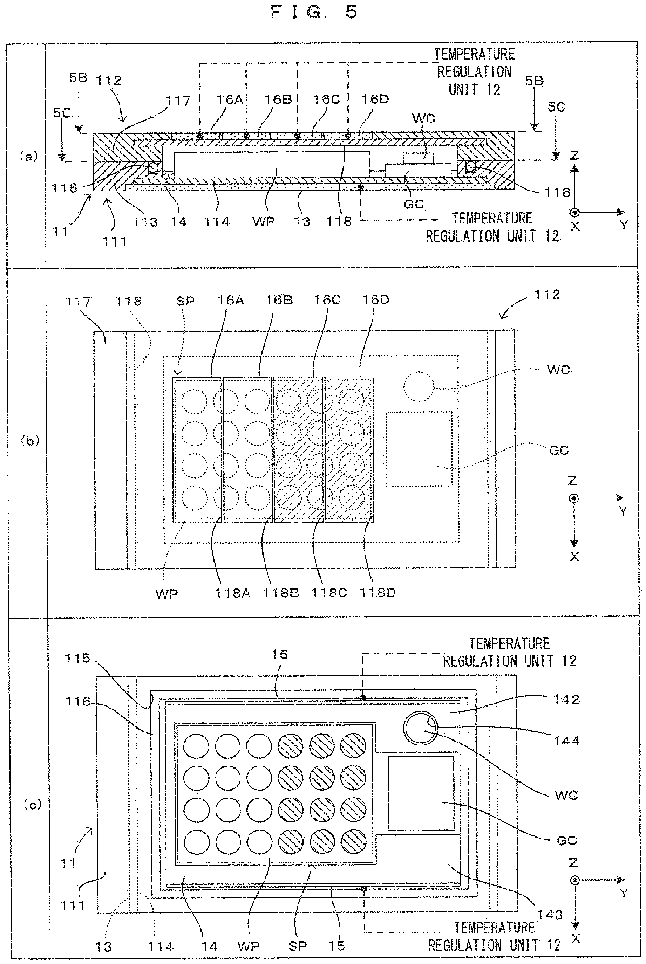

FIG. 5 is a diagram schematically showing the configuration of a third embodiment of the imaging apparatus according to the invention. In FIG. 5, a sectional view showing the structure of an incubator used in the third embodiment is shown in a section (a), a plan view along line 5B-5B indicated by arrows and a plan view along line 5C-5C indicated by arrows in the sectional view are shown in sections (b) and (c). The third embodiment largely differs from the first embodiment in that the upper surface glass heater 16 is divided and used as a plurality of (four in the third embodiment) upper surface glass heaters 16 and the operation and the stop of each of the upper surface glass heaters divided in this way are individually controlled, and the other configuration is basically similar to the first embodiment. Thus, the following description is made, centering on points of difference and the same components and operations are denoted by the same reference signs.

In the third embodiment, recesses 118A to 118D rectangular in a plan view are provided on the upper surface of an upper surface transparent plate 118. The upper surface glass heaters 16A to 16D are respectively fitted into the recesses 118A to 118D and held in close contact with the upper surface transparent plate 118. Further, the upper surface glass heaters 16A to 16D are respectively independently electrically connected to a temperature regulation unit 12, individually generate heat upon receiving power corresponding to a temperature command from a control unit 30 from the temperature regulation unit 12 and partially heat a well plate WP and an accommodation space SP via the upper surface transparent plate 118. Accordingly, states of heat generation of the upper surface glass heaters 16A to 16D can be controlled by individually switching the power feeding and the stop of the power feeding to the upper surface glass heaters 16A to 16D. For example, when biological samples are cultured in 12 wells W on a right half out of 24 wells W as shown in FIG. 5, dew condensation on an upper part of the well plate WP and the upper surface transparent plate 118 becomes problematic only on the right half in observing the samples. Thus, only by causing the upper surface glass heaters 16C, 16D located above the 12 wells W containing the biological samples to generate heat, it is possible to maintain high follow-up observation accuracy while reducing power consumption.

Accordingly, in the third embodiment, a user inputs culture area information relating to the wells W containing the biological samples, i.e. a culture area where the biological samples are cultured via an UI unit 55 and that culture area information is stored in a storage 53 before a follow-up observation. Then, an imaging apparatus performs a time lapse (follow-up observation) in accordance with a follow-up observation program stored in advance in the storage 53.

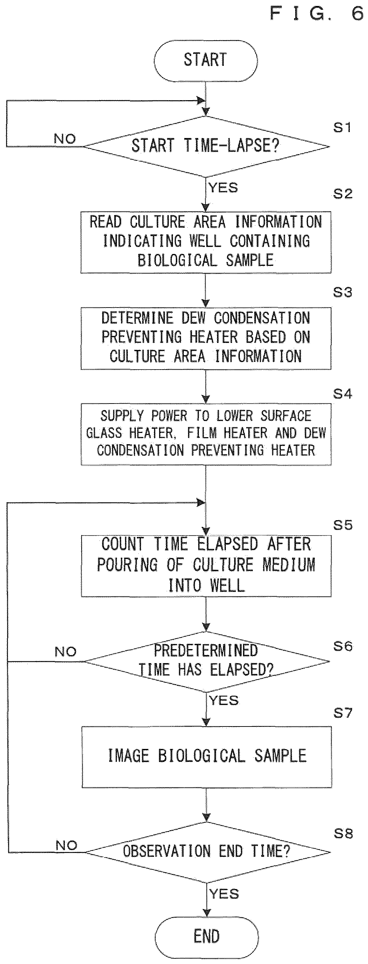

FIG. 6 is a flow chart showing a time-lapse operation by the imaging apparatus of FIG. 5. In the imaging apparatus according to the third embodiment, when a time-lapse start command is given in Step S1, a CPU 51 reads the culture area information from the storage 53 (Step S2) and gives a start command together with the culture area information to the control unit 30.

The control unit 30 discriminates the wells W containing the biological samples based on the culture area information and determines the upper surface glass heater(s) located vertically above, i.e. on a (+Z) direction side of the wells W containing the biological samples (Step S3). In this way, the upper surface glass heaters 16A to 16D are sorted into those requiring power feeding to prevent dew condensation and those not requiring power feeding. For example, in the case shown in FIG. 5, information indicating that the biological samples are contained in 12 wells W on the right half corresponds to the above culture area information and, upon receiving that information, power feeding to the upper surface glass heaters 16C, 16D is judged to be necessary. Note that the upper surface glass heaters exhibiting a dew condensation preventing function by receiving power feeding in this way are called "dew condensation preventing heaters" below.

The control unit 30 sends a temperature command, which is to the effect that power is fed to various heaters except the upper surface glass heaters for which power feeding is judged to be unnecessary, to the temperature regulation unit 12 (Step S4). Then, the temperature regulation unit 12 supplies power to a lower surface glass heater 13 and film heaters 15 and supplies power to the dew condensation preventing heaters (upper surface glass heaters 16C, 16D in FIG. 5) to start temperature regulation and dew condensation prevention in the accommodation space SP.

A culturing process is continued while temperature regulation and dew condensation prevention are performed. The control unit 30 performs an operation of reading the biological samples in the wells W at every predetermined time, e.g. every time 24 hours elapse while counting the time elapsed after the pouring of a culture medium into the wells W (Steps S5 to S7). Specifically, the imaging apparatus performs the reading operation to image the cultured biological samples every time 24 hours elapse until an observation end time is reached.

As described above, since power is supplied to the dew condensation preventing heaters corresponding to the wells W containing the biological samples to prevent dew condensation according to the third embodiment, the follow-up observation can be performed with excellent accuracy without being affected by dew condensation. Further, since the supply of power to the upper surface glass heaters corresponding to the wells W containing no biological samples (upper surface glass heaters 16A, 16B in FIG. 5) is stopped, power saving can be realized. As just described, in the third embodiment, a "dew condensation preventer" of the invention is configured by the temperature regulation unit 12 and the upper surface glass heaters 16A to 16D. Further, the storage 53 corresponds to an example of a "storage unit" of the invention.

Note that although the information relating to the culture area input by the user is read as the culture area information from the storage 53 in the third embodiment, the culture area information may be obtained by performing a pre-scanning operation after the setting of a culture container 11 is completed. Specifically, when the culture container 11 is set in a holder 40 of the imaging apparatus, a light source 29 and an imaging section 21 integrally move within a horizontal plane to image the entire surface of the well plate WP. Since an image obtained in this way includes images of the biological samples, an area of the wells W containing the biological samples, i.e. the culture area where the biological samples are cultured can be obtained as the culture area information based on the imaged image. Note that since a main purpose of pre-scanning is to determine whether or not each well W contains the biological samples, a resolution of pre-scanning may be set at a resolution lower than a resolution during the follow-up observation, e.g. 80[dpi]. As just described, time and labor for the input of the culture area information and an input error can be avoided by performing the pre-scanning operation and a user-friendly and highly reliable imaging apparatus is obtained.

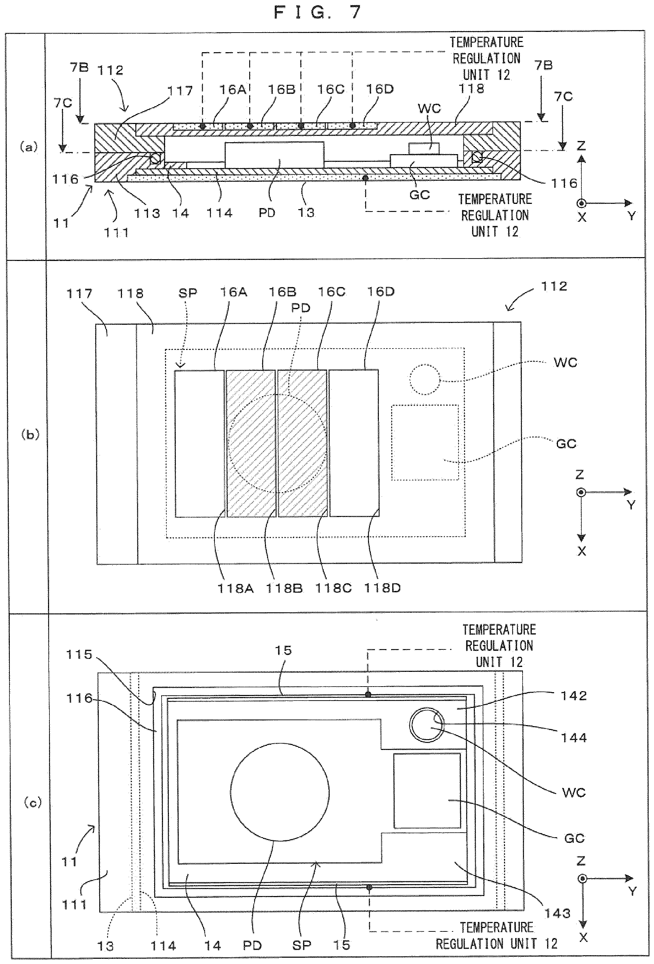

Further, although the follow-up observation is performed using the well plate WP, in which 24 wells W are arranged in a 4x6 matrix as shown in FIG. 5, as a sample carrier in the third embodiment, the number and the arrangement of the wells W are arbitrary without being limited to these. Further, a configuration similar to the third embodiment can be adopted also in the case of performing the follow-up observation by setting a flat plate PD made of transparent glass such as a Schale or a petri dish as the sample carrier in the accommodation space SP, for example, as shown in FIG. 7 (fourth embodiment).

FIG. 7 is a diagram schematically showing the configuration of the fourth embodiment of the imaging apparatus according to the invention. In FIG. 7, a sectional view showing the structure of an incubator used in the fourth embodiment is shown in a section (a), a plan view along line 7B-7B indicated by arrows and a plan view along line 7C-7C indicated by arrows in the sectional view are shown in sections (b) and (c). The fourth embodiment largely differs from the third embodiment in that the flat plate PD is used as the sample carrier, and the other configuration is basically identical. In this fourth embodiment, the flat plate PD is smaller than a well plate WP and, as shown in FIG. 7, a horizontal plane size of the flat plate PD is smaller than a plane area covered by upper surface glass heaters 16A to 16D. When the flat plate PD is set, for example, in a central part of an accommodation space SP, a central area corresponds to a culture area where biological samples are cultured and the upper surface glass heaters 16B, 16C are located vertically above the flat plate PD. In this case, by determining the upper surface glass heaters 16B, 16C as dew condensation preventing heaters, it is possible to perform the follow-up observation with excellent accuracy without being affected by dew condensation while realizing power saving.

Further, although four rectangular divided glass heaters (upper surface glass heaters 16A to 16D) are used in the above third and fourth embodiments, into how many pieces and into which shape the glass heater is divided are arbitrary without being limited to these.

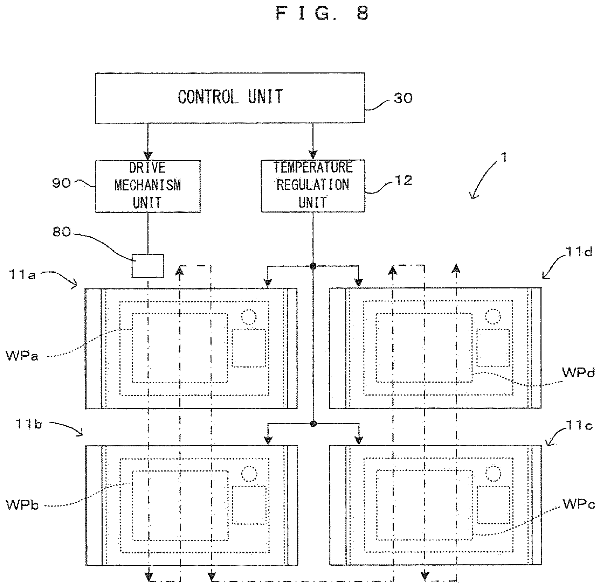

Although one culture container 11 is equipped in the imaging apparatus 100 and the biological samples cultured in the culture environment of the accommodation space SP of the culture container 11 are imaged in the above first to fourth embodiments, imaging is possible in various manners in an imaging system constructed by combining a plurality of culture containers 11 and the imaging apparatus 100. One embodiment of an imaging system according to the invention is described with reference to FIG. 8.

FIG. 8 is a diagram showing one embodiment of the imaging system according to the invention. This imaging system includes four culture containers 11a to 11d and well plates WP1 to WP4 are respectively accommodated into the culture containers 11a to 11d to enable culture. These culture containers 11a to 11d have the same configuration as the culture container 11 of the first embodiment and are arranged in a 2x2 matrix array with upper surface glass heaters 16 faced upward at a predetermined height position. Further, the upper surface glass heaters 16 are electrically connected together with other heaters to a temperature regulation unit 12 and regulate each accommodation space SP to the temperature of a culture environment upon receiving the supply of power. As just described, in this embodiment, an incubator 10 is configured by the four culture containers 11a to 11d and the temperature regulation unit 12, and biological samples can be cultured in parallel in up to four types of culture environments different from each other.

Further, in the imaging system 1, one imaging unit 80 is provided as an "imager" of the invention for the four culture containers 11a to 11d. Although not shown in FIG. 8, the imaging unit 80 includes a light source identically configured to the light source 29 (FIG. 2) of the imaging apparatus 100 and an optical scanning unit identically configured to the optical scanning unit 20 (FIG. 2). Out of these, the light source is arranged in a space above the culture containers 11a to 11d and the optical scanning unit is arranged in a space below the culture containers 11a to 11d, and an imaging section of the optical scanning unit is integrally movable with the light source within a horizontal plane while being located vertically below the light source as in the first embodiment. Note that the imaging unit 80 includes a focusing mechanism for focusing a convergent optical system provided in the imaging section and can adjust a focusing position of an optical image focused on a CCD element.

The imaging unit 80 thus configured is connected to a drive mechanism unit 90. Although not shown, this drive mechanism unit 90 includes an X-drive mechanism for scanning and driving the imaging unit 80 in an X direction and a Y-drive mechanism for scanning and driving the imaging unit 80 in a Y direction, and two-dimensionally moves the imaging unit 80 within the horizontal plane according to a movement command from a control unit 30.

In the imaging system 1 thus configured, the control unit 30 performs a culturing process in the culture containers 11a to 11d and a sample imaging process by the imaging unit 80 by controlling each unit of the apparatus in accordance with a program given in advance. Specifically, the control unit 30 sends a temperature command of each culture container 11a to 11d to the temperature regulation unit 12 upon receiving a start command. Then, the temperature regulation unit 12 supplies power corresponding to the culture environment to each culture container 11a to 11d and starts temperature regulation in the accommodation space SP. By making, for example, supplied power different for each culture container 11a to 11d in this way, the temperatures of the accommodation spaces SP can be made different among the culture containers 11a to 11d, whereby four types of culture environments are obtained.

The culturing process is continued while temperature regulation by the heaters is performed with the culture containers 11a to 11d held in holders (not shown). The control unit 30 gives a drive command to the drive mechanism unit 90 to move the imaging unit 80, for example, as shown by a dashed-dotted line in FIG. 8 at every predetermined time, e.g. every time 24 hours elapse while counting the time elapsed after the pouring of a culture medium into the wells (i.e. sowing of biological tissues and cells). Thus, the imaging unit 80 images the biological samples cultured in the culture container 11a while the light source and the optical scanning unit respectively move while facing the upper and lower surfaces of the culture container 11a. Further, if a movement destination of the imaging unit 80 is switched to another culture container, the biological samples cultured in the culture container as a switching destination are imaged. Note that although each culture container 11a to 11d is scanned a plurality of times since an imaging size of the CCD element of the imaging unit 80 is smaller than a size of the well plates WP in this embodiment, the number of times of scanning may be changed according to a ratio of the imaging size and the plate size.

As described above, according to the imaging system 1, not only the biological samples can be imaged with excellent productivity and at low cost while remaining in the culture environments as in the first embodiment, but also the following functions and effects are obtained. Specifically, in the imaging system 1, each biological sample can be imaged by one imaging unit 80 while the biological samples are cultured in a plurality of culture environments, various usages are possible and high versatility is obtained.

As just described, in the above embodiment, the well plate WP corresponds to an example of the "sample carrier" of the invention. Further, the lower case part 111 and the upper case part 112 respectively correspond to examples of a "base part" and a "ceiling part" of the invention. Further, the lower surface transparent plate 114 and the upper surface transparent plate 118 respectively correspond to examples of a "first transparent part" and a "second transparent part" of the invention. Further, the lower surface glass heater 13 and the upper surface glass heater 16 respectively correspond to examples of a "first transparent electrode" and a "second transparent electrode" of the invention, the film heaters 15 correspond to an example of an "auxiliary temperature regulation unit" of the invention, and these heaters and the temperature regulation unit 12 function as a "temperature regulator" and an "environment regulator" of the invention. Further, the upper surface glass heaters 16A to 16D correspond to "dew condensation preventing transparent electrodes'' of the invention and function as a "dew condensation preventer" of the invention in cooperation with the temperature regulation unit 12. Further, the water container WC corresponds to an example of a "water storage unit'' of the invention and the water container WC and the humidity regulating mechanism 60 function as a "humidity regulator" and an "environment regulator" of the invention. Further, the gas concentration regulating mechanism 70 functions as a "gas concentration regulation unit" and the "environment regulator" of the invention. Further, the inner fixing plate 14 corresponds to an example of a "positioning unit" of the invention. Furthermore, the drive mechanism unit 90 corresponds to an example of a "driver" of the invention.

Note that the invention is not limited to the above embodiments and various changes other than those described above can be made without departing from the gist of the invention. For example, although the temperature in the accommodation space SP is regulated using three types of the heaters 13, 15 and 16 for the culture container 11 in the above embodiments, it is not essential to the invention to use all the three types and it is desirable to provide at least one type out of the three types.

Further, although the temperature regulator, the humidity regulator and the gas concentration regulation unit are provided as the environment regulator for regulating the interior of the accommodation space to the culture environment for the biological samples in the above embodiments, at least one or more may be provided as the environment regulator.

Further, although the lower surface glass heater 13 is arranged on the lower surface side of the lower surface transparent plate 114 and the upper surface glass heater 16 is provided on the upper surface side of the upper surface transparent plate 118 in the above embodiments, at least one of a positional relationship of the lower surface glass heater 13 and the lower surface transparent plate 114 and that of the upper surface glass heater 16 and the upper surface transparent plate 118 may be reversed.

Further, although the four culture containers 11a to 11d are provided in a 2x2 matrix array in the imaging system 1 shown in FIG. 8, the number of the culture containers is not limited to "4" and it is preferable to equip two or more culture containers. Further, an array of a plurality of culture containers is also arbitrary. Further, although the simple incubator shown in FIG. 2 is used as the incubator to be combined with the imaging apparatus 100 in the imaging system 1, it goes without saying that the highly controlled incubator shown in FIG. 4 may also be used.

Further, in the above first to fourth embodiments, a scanning movement is realized by fixing the culture container 11 accommodating the well plate WP and integrally moving the light source 29 and the imaging section 21 relative to the culture container 11. However, a similar scanning movement can be realized also by fixing the light source 29 and the imaging section 21 and moving the culture container 11, and the invention can be applied also to an apparatus having such a configuration.

Further, in the imaging system 1 shown in FIG. 8, a scanning movement is realized by fixing the four culture containers 11a to 11d and moving the imaging unit 80 relative to the culture containers 11a to 11d. However, a similar scanning movement can be realized also by fixing the imaging unit 80 (= light source + optical scanning unit) and moving the culture containers 11a to 11d, and the invention can be applied also to an apparatus having such a configuration.

Further, although the biological samples are imaged by light transmitted through the wells by arranging the light source at the upper side of the culture container(s) 11, 11a to 11d and arranging the optical scanning unit at the lower side in the above embodiments, a positional relationship of the light source and the optical scanning unit is not limited to this. For example, the positional relationship of the light source and the optical scanning unit may be reversed. Further, the biological samples may be imaged by reflected light from the wells. For example, the light source and the optical scanning unit may be arranged at the upper side. In this case, the upper surface glass heater 16 and the upper surface transparent plate 118 respectively function as the "first transparent electrode" and the "first transparent part" of the invention. Further, the light source and the optical scanning unit may be arranged at the lower side. In this case, the lower surface glass heater 13 and the lower surface transparent plate 114 respectively function as the "first transparent electrode" and the "first transparent part" of the invention.

Further, although the transparent plate 114 is arranged in the lower case part 111 in the above embodiments, the entire lower case part 111 may be made of a transparent material and may function as the "first transparent part" of the invention. Similarly to the lower case part 111, the entire upper case part 112 may be made of a transparent material and may function as the "second transparent part" of the invention.

Furthermore, although the well plate(s) WP, WP, to WP4 or the flat plate PD is/are used as the sample carrier(s) in the above embodiments, various other plates are usable as the "sample carrier" of the invention.

### INDUSTRIAL APPLICABILITY

This invention can be particularly preferably applied, for example, in fields requiring the imaging of biological samples such as wells on well plates used in medical and bioscience fields, but its fields of application are not limited to medical and bioscience fields.

### REFERENCE SIGNS LIST

1... date generation system
10...incubator
11, 11a∼11d...culture container
12...temperature regulation unit (dew condensation preventer)
13...lower surface glass heater
14...dinner fixing plate
15...film heater
16...upper surface glass heater
16A ∼ 16D...upper surface glass heater (dew condensation preventing transparent electrode, dew condensation preventer)
20...optical scanning unit
21...imaging section
22...CCD element
29...light source
30...control unit
60...humidity regulating mechanism
61...humidify sensor
62...humidity regulation unit
63...steam supply unit
70...gas concentration regulating mechanism
71...carbon dioxide concentration sensor
72...carbon dioxide concentration regulation unit
73...carbon dioxide cylinder
74...gas supply unit
80...imaging unit
90...drive mechanism unit (driver)
100...imaging apparatus
111...lower case part
112... upper case part
114...slower surface transparent plate
118...upper surface transparent plate
GC... gas concentration regulator
PD...flat plate (sample carrier)
W...well
WC...water container

## Claims

1. An imaging apparatus, comprising:
a culture container including an accommodation space for accommodating a sample carrier carrying biological samples in a culture environment for the biological samples and a first transparent part making the accommodation space observable from outside; and
an imager that images the biological samples in the accommodation space via the first transparent part.

2. The imaging apparatus according to claim 1, further comprising a dew condensation preventer, wherein:
the first transparent part is arranged above the sample carrier accommodated in the accommodation space; and
the dew condensation preventer controls power feeding to each of a plurality of dew condensation preventing transparent electrodes provided in the first transparent part according to a culture area where the biological samples are cultured.

3. The imaging apparatus according to claim 1, further comprising a dew condensation preventer, wherein:
the culture container includes a second transparent part arranged above the sample carrier accommodated in the accommodation space for illuminating the biological samples by guiding light to the accommodation space from outside of the culture container; and
the dew condensation preventer controls power feeding to each of a plurality of dew condensation preventing transparent electrodes provided in the second transparent part according to a culture area where the biological samples are cultured.

4. The imaging apparatus according to claim 2 or 3, further comprising a storage unit that stores culture area information relating to the culture area, wherein:
the dew condensation preventer controls power feeding to each dew condensation preventing transparent electrode based on the culture area information stored in the storage unit.

5. The imaging apparatus according to claim 2 or 3, wherein:
the imager images the sample carrier accommodated in the accommodation space before the biological samples are cultured; and
the dew condensation preventer controls power feeding to each dew condensation preventing transparent electrode based on culture area information relating to the culture area and included in an image imaged by the imager.

6. The imaging apparatus according to claim 1, further comprising a temperature regulator that regulates a temperature in the accommodation space to the culture environment.

7. The imaging apparatus according to claim 6, wherein:
the temperature regulator includes a first transparent electrode provided in the first transparent part and regulates the temperature in the accommodation space by feeding power to the first transparent electrode and causing the first transparent electrode to generate heat.

8. The imaging apparatus according to claim 6 or 7, wherein:
the culture container includes a second transparent part for illuminating the biological samples by guiding light to the accommodation space from outside of the culture container; and
the temperature regulator includes a second transparent electrode provided in the second transparent part and regulates the temperature in the accommodation space by feeding power to the second transparent electrode and causing the second transparent electrode to generate heat.

9. The imaging apparatus according to claim 8, wherein:
the culture container includes a lower case part for supporting the sample carrier from below and an upper case part arranged above the sample carrier supported in the lower case part and forms the accommodation space by integrating the lower and upper case parts; and
the first transparent part and the first transparent electrode are provided in one of the lower and upper case parts and the second transparent part and the second transparent electrode are provided in the other.

10. The imaging apparatus according to claim 9, wherein:
the temperature regulator independently controls each of a power value to be given to the first transparent electrode and a power value to be given to the second transparent electrode.

11. The imaging apparatus according to claim 9 or 10, wherein:
the temperature regulator includes an auxiliary temperature regulation unit provided between the lower and upper case parts for regulating the temperature of the accommodation space.

12. The imaging apparatus according to any one of claims 1 to 11, comprising a humidity regulator that regulates a humidity in the accommodation space to the culture environment.

13. The imaging apparatus according to claim 12, wherein:
the humidity regulator includes a water storage unit for storing water; and
the water storage unit is arranged in the culture container with the stored water exposed to the accommodation space.

14. The imaging apparatus according to claim 12, wherein:
the humidity regulator includes a steam supply unit that supplies steam to the accommodation space.

15. The imaging apparatus according to any one of claims 1 to 14, further comprising a gas concentration regulation unit for regulating a carbon dioxide concentration in the accommodation space to the culture environment.

16. The imaging apparatus according to claim 15, wherein:
the gas concentration regulation unit includes a positioning unit that positions gas concentration regulator for regulating a carbon dioxide concentration by generating carbon dioxide with the gas concentration regulator exposed to the accommodation space.

17. The imaging apparatus according to claim 15, wherein:
the gas concentration regulation unit includes a gas supply unit that supplies the carbon dioxide to the accommodation space.

18. An imaging system, comprising:
a plurality of culture containers each including an accommodation space for accommodating a sample carrier carrying biological samples in a culture environment for the biological samples and a first transparent part making the accommodation space observable from outside;
an imager that images the biological samples; and
a driver that switches the culture container facing the imager by relatively moving the imager with respect to the plurality of culture containers; wherein
the imager images the biological samples in the accommodation space via the first transparent part of the culture container facing the image every time the culture container facing the imager is switched.

19. The imaging system according to claim 18, further comprising a dew condensation preventer, wherein:
the first transparent part is arranged above the sample carrier accommodated in the accommodation space in each culture container; and
the dew condensation preventer controls power feeding to each of a plurality of dew condensation preventing transparent electrodes provided in the first transparent part for each culture container according to a culture area where the biological samples are cultured.

20. The imaging system according to claim 18, further comprising a dew condensation preventer, wherein:
each culture container includes a second transparent part arranged above the sample carrier accommodated in the accommodation space for illuminating the biological samples by guiding light to the accommodation space from outside of the culture container; and
the dew condensation preventer controls power feeding to each of a plurality of dew condensation preventing transparent electrodes provided in the second transparent part for each culture container according to a culture area where the biological samples are cultured.

21. The imaging system according to any one of claims 18 to 20, wherein:
the plurality of culture containers are fixedly arranged, whereas the driver switches the culture containers by moving the imager.

22. An incubator for imaging biological samples carried in a sample carrier by an imager of an imaging apparatus in a state where the biological samples are cultured, the incubator comprising:
a culture container including an accommodation space for accommodating the sample carrier; and
an environment regulator that regulates the interior of the accommodation space to a culture environment for the biological samples; wherein
the culture container is provided separately from the imager and including a first transparent part making the accommodation space observable from outside of the culture container.

23. The incubator according to claim 22, wherein:
the first transparent part is arranged above the sample carrier accommodated in the accommodation space; and
power feeding to each of a plurality of dew condensation preventing transparent electrodes provided in the first transparent part is controlled according to a culture area where the biological samples are cultured.

24. The incubator according to claim 23, wherein:
the culture container includes a second transparent part arranged above the sample carrier accommodated in the accommodation space for illuminating the biological samples by guiding light to the accommodation space from outside of the culture container; and
power feeding to each of a plurality of dew condensation preventing transparent electrodes provided in the second transparent part is controlled according to the culture area where the biological samples are cultured.

25. The incubator according to claim 22, wherein:
the environment regulator includes a first transparent electrode provided in the first transparent part and regulates a temperature in the accommodation space to the culture environment by feeding power to the first transparent electrode and causing the first transparent electrode to generate heat.

26. The incubator according to claim 22 or 25, wherein:
the culture container includes a second transparent part for illuminating the biological samples by guiding light to the accommodation space from outside of the culture container; and
the environment regulator includes a second transparent electrode provided in the second transparent part and regulates a temperature in the accommodation space to the culture environment by feeding power to the second transparent electrode and causing the second transparent electrode to generate heat.
